# EUROPEAN PATENT SPECIFICATION

(11) **EP 2 029 028 B1**
(45) Date of publication and mention of the grant of the patent: **10.07.2013**
(21) Application number: 07777096.4
(22) Date of filing: 15.05.2007
(51) Int. Cl.: A61B 17/00

(54) **PATENT FORAMEN OVALE CLOSURE DEVICE AND METHOD**
VERSCHLUSSVORRICHTUNG FÜR EIN OFFENES FORAMEN OVALE UND ENTSPRECHENDES VERFAHREN
DISPOSITIF ET PROCÉDÉ DE FERMETURE DU FORAMEN OVALE PERMÉABLE

(30) Priority: 18.05.2006 US 801637 P
(43) Date of publication of application: 04.03.2009
(73) Proprietor: Cook Medical Technologies LLC, Bloomington, IN 47404 (US)
(72) Inventor: OSBORNE, Thomas, A., Bloomington, IN 47401 (US)
(74) Representative: Garratt, Peter Douglas
(86) International application number: PCT/US2007/011743
(87) International publication number: WO 2007/136660

(56) References cited:
- EP-A- 1 222 897
- WO-A-93/13712
- WO-A-03/101312
- WO-A-2005/034738
- GB-A- 2 269 321
- US-A1- 2002 183 787
- US-A1- 2004 073 242
- US-B1- 6 270 515

## Description

### Technical Field

The present invention relates to a device for closure of a patent foramen ovale (PFO). More particularly, the invention relates to a patent foramen ovale closure device utilizing an expandable frame member suitable for percutaneous introduction.

### Background of the Invention

In the fetal heart, there is a small communication, referred to as the foramen ovale, in the septum between the right and left atria. In the unborn fetus, this communication allows blood to bypass the lungs. Fetal blood is oxygenated by the lungs of the mother. This communication normally closes within the first year after birth, and oxygenation is carried out through the baby's own lungs. Although the remnant of the opening remains in the septum after birth, the remnant normally does not allow passage of blood.

In some cases, however, this opening (the foramen ovale) remains patent and the baby's oxygenated blood is diluted by un-oxygenated venous blood. Babies with this condition often have very little energy, are cyanotic (blue coloration), and do not progress well after birth. In recent years, physicians have also discovered that in a large percentage of adults, estimated at about 30%, the foramen ovale has not completely sealed, and remains as a small patent foramen ovale. In these adults, there is still some leakage across the septum through the remnant foramen ovale. Although such leakage is not always problematic, the leakage can be aggravated upon certain types of strain or valsalva. Intermittent leakage of blood through the PFO has been linked to migraine headaches and other maladies. In addition, a PFO is suspected as being a passageway for blood clots. Passage of clots through the opening can lead to a stroke or a transient ischemic attack (TIA).

An atrial septal defect (ASD) is a definable hole that extends through the septum of newborns. The leaking, or patent, foramen ovale does not result from the same physiological structure as an ASD. An ASD can be occluded by passing known occluder devices through the hole, such that the devices anchor on each side of the septum to form a seal. Current devices that are commonly used for ASD repair include the Amplatzer ASD Occluder, available from AGA Medical, and the Gianturco occluder coils, available from Cook Incorporated.

Unlike the definable hole that forms an ASD, the foramen ovale is a small channel or slot-type structure that is defined by the septum and a flap that covers a part of the ovale. With a PFO, the septum and the flap normally overlap to a certain degree, and are not fused together as in the normal case. This permits the leakage of small amounts of blood through the channel that extends between the septum and the flap.

Currently available ASD repair devices are ill suited for repair of a PFO. As stated, ASD repair devices normally comprise an occluder-type structure that is extended through the septum hole that comprises the defect to seal the opening. However, with a PFO, the openings on each side of the septum are offset, and not in line with each other (i.e., not directly across from each other). The leakage path is under a flap, and through a narrow channel, rather than a defined hole. Thus, it is not generally sufficient to merely provide a plug for a hole, as in conventional ASD repair.

Open heart surgical methods have been used for PFO repair. Such methods normally entail opening the chest cavity, and cutting into the heart muscle. The flap is then sutured or otherwise attached to the septum, in a manner such that the passageway is closed. Although generally effective for closing the PFO, such methods are intrusive, costly, and require an extended recovery period for the patient.

Recently, percutaneous methods have been developed for repair of a PFO. These methods involve utilizing conventional percutaneous entry techniques, and thereafter passing a catheter through a vessel into the right atrium of the heart. An occluder device, such as the Amplatzer PFO Occluder, is passed through the catheter and positioned in the opening. This device comprises a plug-like device formed of a self-expanding wire-mesh with double discs. The device contains inner polyester fabric patches that, along with the wire mesh, are intended to cause the formation and accumulation of a blood clot. The resulting blood clot is positioned to block the opening. Devices of this type are complex mechanically, require a high level of skill to insert properly, and result in the formation of a clot which actually forms the seal.

WO 03/101312 discloses an intracordiac occluder including a proximal support structure supporting the proximal occlusion shell and a distal support structure supporting the distal occlusion shell. The shells may be formed from biological tissue derived from the tunica submucosa layer of the porcine small intestine.

There exists a need for a device for providing effective closure of a patent foramen ovale, which device is suitable for percutaneous entry, is less complex mechanically and operationally when compared to prior art devices, and which can be utilized for patent foramen ovale closure with minimal trauma to the patient.

### Summary of the Intention

The present invention addresses the shortcomings of the prior art. The invention comprises a device for closure of a patent foramen ovale of a patient as defined in claim 1.

### Brief Description of the Drawing

Fig. 1 is an illustration of the internal portions of the heart;
Fig. 1A is an enlarged view of a portion of the heart shown in Fig. 1, illustrating the location of a patent foramen ovale;
Fig. 2 is a side view of a patent foramen ovale closure device according to an embodiment of the present invention, shown in its expanded condition;
Fig. 3 is an enlarged sectional view of the device in the expanded condition, taken along lines 3--3 of Fig. 2;
Fig. 4 is a side view of the patent foramen ovale closure device of Fig. 2, shown in its collapsed condition;
Fig. 5 is an enlarged sectional view of the device in the collapsed condition, taken along lines 5--5 of Fig. 4;
Figs. 6 and 7 are enlarged sectional views of alternative versions of the device in the expanded condition;
Fig. 8 is a side view of an alternative embodiment of a patent foramen ovale closure device, shown in its expanded condition;
Fig. 9 is a side view of another alternative embodiment of a patent foramen ovale closure device in its expanded condition;
Figs. 10 and 10A are side views of further alternative embodiments of the device in its expanded condition; and
Figs. 11 and 12 are side views of alternative frame members for use with the device.

### Detailed Description

For the purposes of promoting an understanding of the principles of the invention, reference will now be made to the embodiments illustrated in the drawings, and specific language will be used to describe the same. It should nevertheless be understood that no limitation of the scope of the invention is thereby intended, such alterations and further modifications in the illustrated device, and such further applications of the principles of the invention as illustrated therein being contemplated as would normally occur to one skilled in the art to which the invention relates.

The present invention relates to a device for closure of a patent foramen ovale. In the following discussion, the terms "proximal" and "distal" will be used to describe the opposing axial ends of device, as well as the axial ends of various component features of the device. The term "proximal" is used in its conventional sense to refer to the end of the device (or component) that is closest to the operator during use of the device. The term "distal" is used in its conventional sense to refer to the end of the device (or component) that is initially inserted into the patient, or that is closest to the patient.

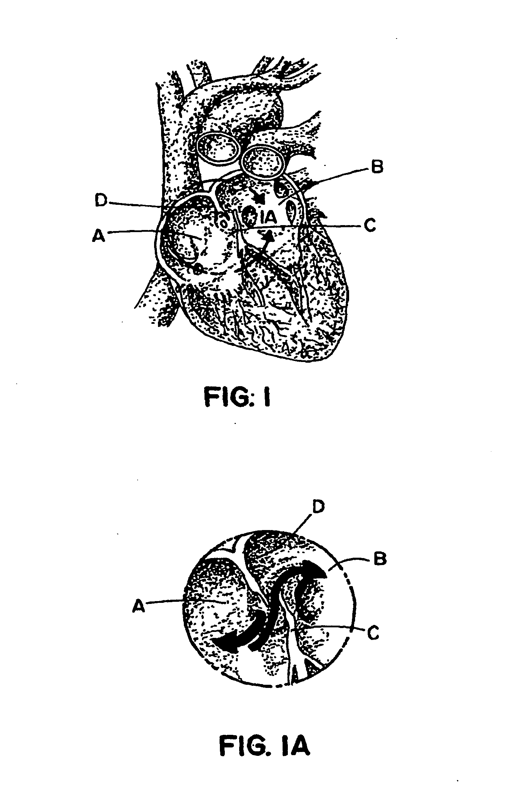

Fig. 1 is an illustration of the internal portions of the heart, showing the right atrium A, left atrium B, septum C that separates the right and left atria, and flap D that extends along a portion of the septum C. Fig. 1A is an enlarged view of a portion of the heart shown in Fig. 1. As shown in Fig. 1A, septum C and flap D define a channel (shown by the arrows in Fig. 1 A) therebetween that comprises the patent foramen ovale. The presence of the PFO establishes a communication between the atria. This communication allows blood to leak between the chambers, which leakage can result in the migration of unoxygenated blood from the left atrium to mix with the oxygenated blood in the right atrium. Such leakage has been linked to migraine headaches and other maladies described above, such as a stroke or a transient ischemic attack (TIA).

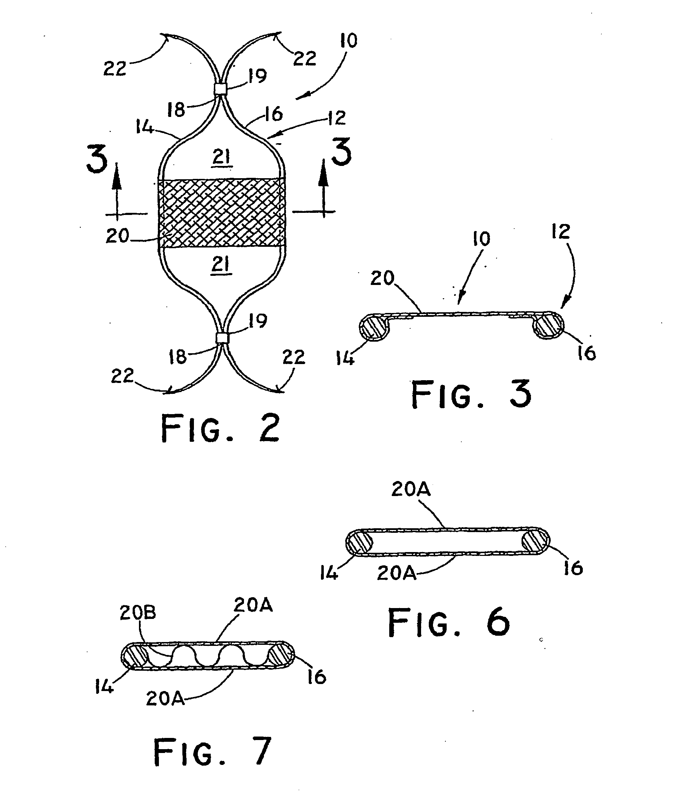

Fig. 2 illustrates a side view of a patent foramen ovale closure device 10 according to one embodiment of the present invention. Fig. 3 illustrates an enlarged sectional view of the device, taken along lines 3--3 of Fig. 2. Device 10 comprises a planar frame member 1 2, formed from adjoining wire members 14, 16. Frame member 12 carries a cover material 20 suitable for promoting clot formation upon insertion of the device into the foramen ovale of a patient, and/or for promoting tissue growth between the cover material and surrounding tissue. In the embodiment shown, covering material 20 spans an opening 21 between wire members 14, 16. Wire members 14, 16 are joined near their respective axial ends at one or more joinder points 18 by conventional means, such as soldering, welding, brazing, and the like. Suitable anchoring devices, such as hooks 22, may be provided at the axial ends of each wire member 14, 16 for anchoring the device to the opposing flaps in the heart that define the foramen ovale.

If desired, one or more radiopaque markers 19 may be incorporated into the structure of the device to improve visibility under conventional medical imaging techniques, such as x-ray fluoroscopy. The use of radiopaque markers is well known in the medical arts, and those skilled in the art can readily select an appropriate marker for a particular use. Radiopaque markers formed from metals such as tungsten, platinum or gold are particularly preferred for use with frame member 12. Such metals can be conveniently supplied in the form of bands, and can be applied to the device, e.g., at joinder points 18 where the wires meet. In a preferred embodiment, the bands are positioned over the wires, and become part of the joint formed by wires 14, 16.

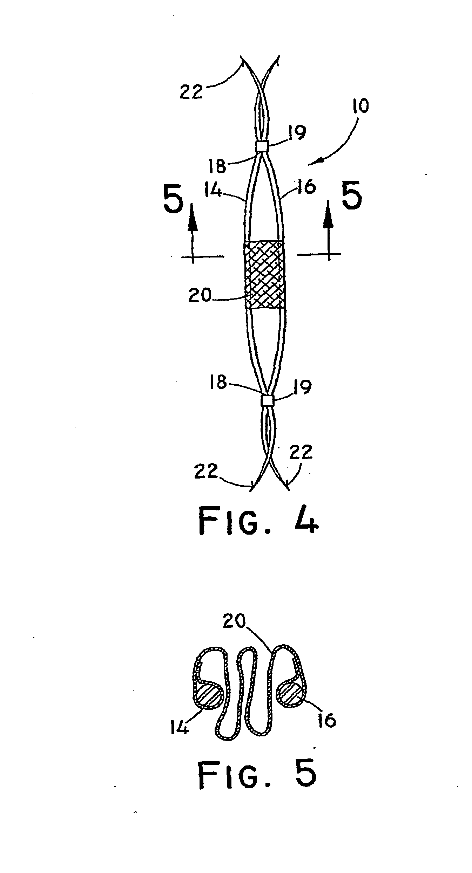

Device 10 is selectively movable between the expanded condition shown in Fig. 2, and a collapsed condition as shown in Fig. 4. The device is sized such that when it is in the collapsed condition shown in Fig. 4, it is receivable in a delivery sheath of a type suitable for percutaneously delivering the device for placement in the heart. Fig. 5 illustrates an enlarged sectional view of the device when in the collapsed condition, taken along lines 5--5 of Fig. 4.

Figs. 6 and 7 show enlarged sectional views of alternative arrangements of the covering material of the device, taken from the same perspective as Fig. 3. In the embodiment of Fig. 6, the covering material 20A is wrapped around wires 14, 16, such that two layers of covering material are provided. In the embodiment of Fig. 7, the covering material comprises two wrapped-around layers 20A as in Fig. 6, with a third layer 20B filling a portion of the space between the two outer layers. The presence of the additional covering material in Figs. 6 and 7 may further enhance the clot and/or tissue growth potential of the covering material when positioned in the foramen ovale.

Preferably, wire members 14, 16 are formed of a biologically compatible material that is capable of self-expanding upon deployment from the delivery sheath, and maintaining its shape and location until the device becomes endothelialized, or incorporated into the surrounding tissue. Self-expandable materials of this type are well known in the medical arts, and include, among others, spring tempered stainless steel, nitinol in the super-elastic state, and palladium.

The covering material may be formed from one or more components that are suitable for promoting the desired activity of the material. For example, it may be desired to close the foramen ovale by forming a clot within the ovale. In this event, suitable covering materials may include compositions known in the medical arts for promoting clot formation, such as polyester and silk fibers, among others. One particularly suitable covering material comprises DACRON^{®} fibers.

Alternatively, it may be desired to close the foramen ovale by providing a cover material that spans the ovale and is capable of growing into the surrounding tissue at each side of the ovale. In this event, suitable covering materials may include growth-promoting compositions. In a preferred embodiment, the growth promoting material is bioremodelable. A bioremodelable material can provide an extracellular matrix that permits, and may even promote, cellular invasion and ingrowth into the material upon implantation. Non-limiting examples of bioremodelable materials include reconstituted or naturally-derived collagenous materials. Preferably, the material is an extracellular matrix material (ECM) possessing biotropic properties, including in certain forms, angiogenic collagenous extracellular matrix materials. For example, suitable collagenous materials include ECMs such as submucosa, renal capsule membrane, dermal collagen, dura mater, pericardium, fascia lata, serosa, peritoneum or basement membrane layers, including liver basement membrane. Suitable submucosa materials for these purposes include, for instance, intestinal submucosa, including small intestinal submucosa, stomach submucosa, urinary bladder submucosa, and uterine submucosa. The submucosa or other ECM material used in the present invention may also exhibit an angiogenic character and thus be effective to induce angiogenesis in a host engrafted with the material.

Suitable bioremodelable material having in vivo angiogenic properties may be identified using a subcutaneous implant model to determine the angiogenic character of a material, as disclosed in C. Heeschen et al., Nature Medicine 7 (2001), No. 7, 833-839. When combined with a fluorescence microangiography technique, this model can provide both quantitative and qualitative measures of angiogenesis into biomaterials. C. Johnson et al., Circulation Research 94 (2004), No. 2, 262-268. Submucosa or other ECM materials of the present invention can be derived from any suitable organ or other tissue source, usually sources containing connective tissues. Submucosa or other ECM tissue used in the invention is preferably highly purified, for example, as described in U.S. Patent No. 6,206,931 to Cook et al.

Such covering material may include a bioactive component that induces, directly or indirectly, a cellular response such as a change in cell morphology, proliferation, growth, protein or gene expression. For example, the submucosa material and any other ECM used may also optionally retain growth factors or other bioactive components, such as basic fibroblast growth factor (FGF-2), transforming growth factor beta (TGF-beta), epidermal growth factor (EGF), and/or platelet derived growth factor (PDGF). Further, in addition or as an alternative to the inclusion of native bioactive components, non-native bioactive components such as those synthetically produced by recombinant technology or other methods, may be incorporated into the submucosa or other ECM tissue, including drug substances such as antibiotics or thrombus-promoting substances such as blood clotting factors, e.g. thrombin, fibrinogen, and the like. These substances may be applied to the ECM material as a premanufactured step, immediately prior to the procedure (e.g. by soaking the material in a solution containing a suitable antibiotic such as cefazolin), or during or after engraftment of the material in the patient.

In addition to the foregoing, the covering material can include compositions for promoting both clot formation and tissue growth.

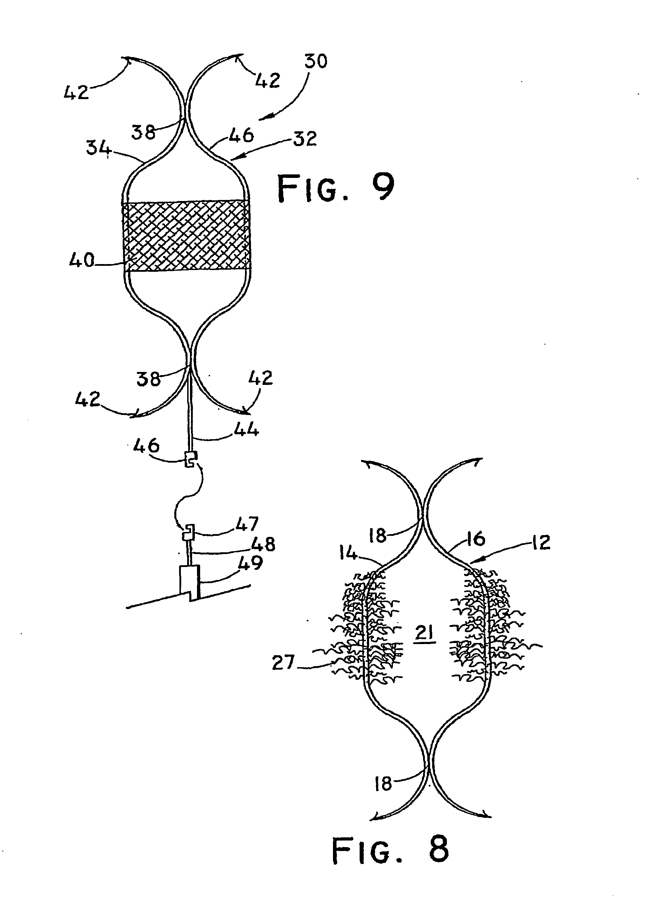

Although the embodiments illustrated in Figs. 2-7 include a covering material 20 that spans an opening 21 between wire members 14, 16 when in the expanded condition, this arrangement is not required, and other arrangements may be substituted. One alternative arrangement is illustrated in Fig. 8. In this embodiment a frame member 12 is formed from wire members 14, 16 as before, which wire members may be joined at joinder points 18. However, rather than including a covering material that spans opening 21, a plurality of clot and/or growth promoting fibers 27 are tied or otherwise adhered to the respective wire members. Typically, in a situation when fibers are tied to a frame as described, the fibers will comprise clot promoting fibers. Although the fibers may be tied to any portion of the frame, it is preferred to tie the fibers to the same general portions of the wire members that had been engaged with the covering material in the previous embodiment. Those skilled in the art will appreciate that any number, and size, of fibers may be tied or otherwise engaged with the frame. In one preferred embodiment, there will be a sufficient number of fibers to cover the length of the frame otherwise covered by material 20, and the fibers will have a length of about 2-4 mm. If desired, one or more radiopaque markers, such as marker 19 shown in Fig. 2, may also be included.

A suitable delivery system for the inventive PFO closure may comprise a conventional delivery sheath having a length sufficient to extend from an entry vessel, such as the femoral vein in the groin area, through the inferior vena cava into the right ventricle of the heart, and ultimately into the PFO. Typically, the delivery sheath will have a length of about 80-100 cm. Non-limiting examples of suitable delivery sheaths include conventional PTFE sheaths, as well as FLEXOR^{®} sheaths, available from Cook Incorporated, of Bloomington, IN. The delivery sheath may be introduced by conventional means, such as the well-known Seldinger percutaneous entry technique. This technique is commonly used for accessing the right atrium of the heart. In the Seldinger technique, a puncture is made by injecting a needle into the entry vessel. A wire guide is then inserted through a bore in the needle into the vessel, and the needle is thereafter withdrawn. The wire guide is threaded into the right atrium of the heart, and a delivery sheath is threaded over the wire guide into the atrium. Following proper placement of the delivery sheath, the wire guide may be withdrawn in conventional fashion.

Once the sheath has been positioned across the PFO, the PFO closure device 10 can be advanced through the sheath utilizing a conventional pusher until it is properly positioned (while still inside the sheath) across the PFO. Preferably, the positioning of the closure device 10 is established by radiopaque markers and fluoroscopy. Upon confirmation of proper placement, the sheath may be withdrawn slightly while device 10 and the pusher are maintained in a stationary position. When device 10 is fully unsheathed, it will expand and occlude the PFO. The delivery sheath may then be fully withdrawn.

Fig. 9 illustrates an embodiment of a PFO closure device 30 suitable for use with one embodiment of a delivery device. With the exception of the additional structure for use with the delivery device, device 30 may otherwise be similar to device 10. That is, device 30 may comprise a planar frame member 32, formed from adjoining wire members 34, 36, which wire members may be joined near their respective axial ends at one or more joinder points 38. Frame member 32 may be provided with cover material 40 (or fibers 27) for promoting clot and/or tissue growth formation. Suitable anchoring devices 42 and/or radiopaque markers (not shown) may be provided at the axial ends of each of wire members 34, 36.

In the embodiment of Fig. 9, closure device 30 is provided with an extensible portion 44 at its proximal end, which extensible portion terminates in a coupler 46. Coupler 46 is structured and arranged for easy coupling, and uncoupling, with a mating coupler 47 at the distal end of a conventional pusher or positioning wire guide 48. Pusher or wire guide 48 is deployable from the distal end of a delivery sheath 49 in normal fashion. Typically, couplers 46, 47 remain coupled when device 30 is housed within the delivery sheath, and uncouple once the closure device has been advanced to the desired position outside of the delivery sheath.

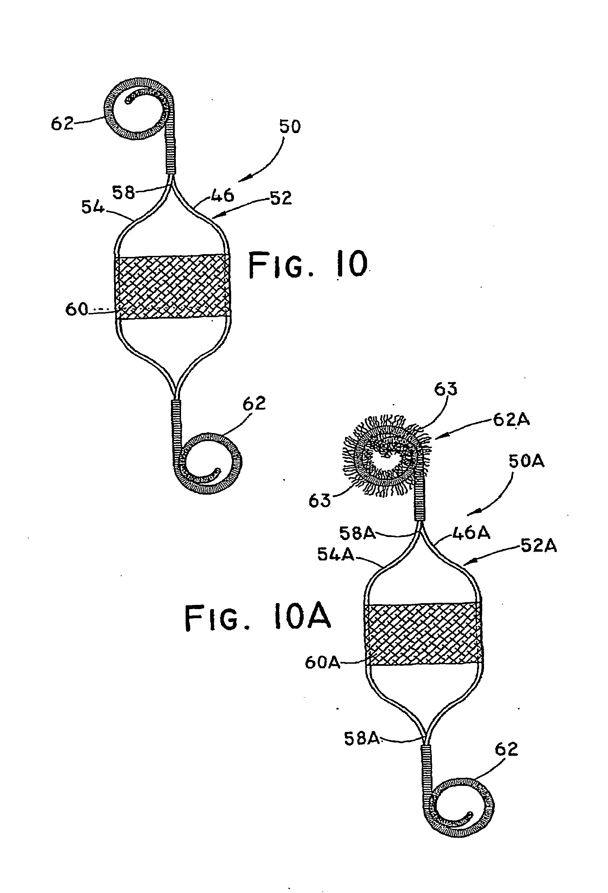

Following an initial deployment of the closure device, placement of the device is observed utilizing a suitable visualization technique, such as fluoroscopy. If the initial placement is deemed unsatisfactory, couplers 46, 47 can be recoupled, and the device can be retracted into the delivery system. Couplers 46, 47 capable of re-coupling are known in the art and may include, for example, a mating tongue and groove construction, or an arrangement comprising mating screw threads on the respective couplers. Such mating screw threads may be formed by simply stretching the coils of a wire guide to form the mating threads. Following re-coupling and retraction into the delivery sheath, the closure device is re-deployed and the visualization process is repeated. Fig. 10 illustrates an alternative embodiment of a PFO closure device 50. Device 50 comprises a planar frame member 52 formed from wire members 54, 56 that are joined at joinder points 58, as before. Frame member 52 may be provided with a cover material 60 for promoting clot formation. In this embodiment, instead of the anchoring hooks of the previous embodiments, wire guide coils 62 have been provided at each axial end of the device to anchor the device in the foramen ovale. Wire guide coils .62 may be attached to frame member 52 in any conventional fashion, such as by sliding the coils over the respective axial ends of the frame member, and thereafter affixing the coils to the frame in any well-known fashion, such as by soldering, brazing, welding, etc. Typically, the coils are formed from a material, such as spring tempered cold-worked stainless steel, having a memory such that the coils may be initially deployed from the delivery sheath in linear fashion, and thereafter re-form into a coil following deployment. The coils are sized such that upon deployment in the foramen ovale, they comprise a mass of a size that is not thereafter movable through the opening under normal conditions encountered within the interior spaces of the heart.

Fig. 10A illustrates still another alternative embodiment of a PFO closure device. Device 50A is similar to device 50 of Fig. 10, with the exception of a fibered wire coil 62A that may be substituted for coil 62. Fibered wire coil 62A may be provided at one (as shown in Fig. 10A), or both axial ends of the PFO closure device. Fibers 63 may be of conventional size and construction, and may be similar to fibers 27 described previously. As stated, the presence of the fibers may be particularly beneficial for promoting clot formation.

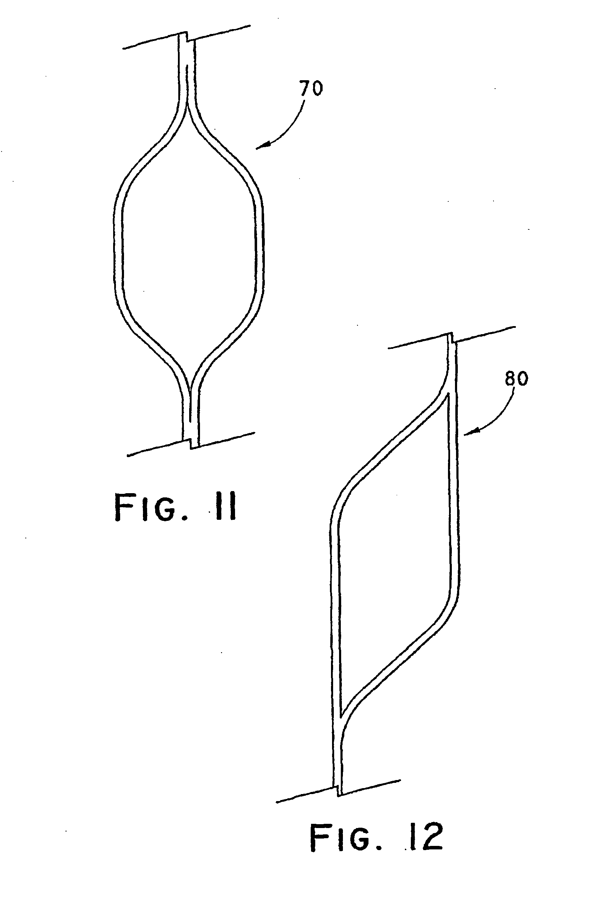

Figs. 11 and 12 illustrate additional alternative designs of a planar frame member suitable for use in the inventive closure device. In Fig. 11, frame member 70 is of a type that may be obtained by cutting or otherwise excising the frame member from a flat sheet of material. The material may be any of the self- expandable materials described previously for use as the frame members, such as spring tempered stainless steel, nitinol in the super-elastic state, and palladium. In this case, however, the frame member may simply be cut from a sheet in conventional fashion, such as by laser cutting, or retrieved from a sheet in some other conventional manner, such as by photo etching the sheet. The frame may be cut, etched, etc., into any desired configuration, including but not limited to, the preferred configuration illustrated in Fig. 2.

Frame member 80 of Fig. 12 may also be cut, etched, etc., from a sheet in the same manner as the frame member of Fig. 11. In this variation, frame member 80 is configured to allow some tilting of the device when positioned in the PFO. It is believed that permitting the device to tilt in the PFO may allow the device to be more tolerant of variations in sizes of a PFO, and to thereby more readily conform to such variations.

Although not shown in Figs. 11 and 12, frame members 70, 80 may also be provided with radiopaque markers and anchoring devices as before. Similarly, suitable growth-promoting materials, such as the clot forming or tissue growth materials described above, may be provided at desired locations along the frame member.

Although the present invention has been described with reference to its preferred embodiment as a device for closure of a patent foramen ovale, the invention is not so limited. Rather, the inventive device can be utilized for closure of other small channels or passageways encountered between adjacent tissue borders within the body of a patient.

While these features have been disclosed in connection with the illustrated preferred embodiments, other embodiments of the invention will be apparent to those skilled in the art that come within the scope of the invention as defined in the following claims.

## Claims

1. A device (10) for closure of a patent foramen ovale of a patient, the device having opposing axial ends, comprising: a frame member (12) having a collapsed condition and an expanded condition in which the frame member is planar, the frame member comprising a pair of elongated members (14, 16) each defining a first and a second axial and, said elongated members joined near said first axial ends (18) at a distal joinder point and near said second axial ends (18) at a proximal joinder point, said frame is such that said,
elongated members are positioned substantially adjacent one another when the frame member is in the collapsed condition, and are positioned such that an opening is defined therebetween when the frame member is in the expanded condition, the frame member sized and arranged to be percutaneously insertable into an area of the foramen ovale when in the collapsed condition, and to substantially span the foramen ovale when in the expanded condition;
a promoter (20, 27) carried by the frame member, the promoter capable of promoting biological formation to effect a closure of the foramen ovale; and
an anchoring member (22, 62) at each axial end of the elongated members configured to anchor the frame member to tissue adjacent the frame member when the frame member is in the expanded condition.

2. The device of claim 1, said promoter comprising a covering material, a first portion of the covering material engaged with one of the elongated members and a second portion of the covering material engaged with the other elongated member, the covering material positioned to substantially span the opening when the frame member is in the expanded condition.

3. The device of claim 2, wherein the covering material comprises a composition capable of promoting clot formation in the foramen ovale.

4. The device of claim 3, wherein the clot-promoting composition comprises a fibrous material.

5. The device of claim 4, wherein the fibrous material comprises polyester or silk fibers.

6. The device of claim 2, wherein the covering material comprises a tissue growth-promoting composition capable of promoting growth with tissue adjacent the foramen ovale.

7. The device of claim 6, wherein the tissue growth-promoting composition comprises at least one of a small intestine submucosa and a collagen-based material.

8. The device of claim 1, wherein the anchoring member comprises at least one hook capable of anchoring to the adjacent tissue.

9. The device of claim 1, wherein the anchoring member comprises at least one coil sized and configured for anchoring the device in the foramen ovale.

10. The device of claim 9, wherein said coil includes a plurality of clot promoting fibers engaged thereto.

11. The device of claim 1, further comprising at least one radiopaque marker engaged with the frame member.

12. The device of claim 1, the promoter comprising a plurality of clot-promoting fibers engaged with the frame members.

13. The device of claim 12, the fibers being engaged with at least one of the frame members between said joinder points.

14. The device of claim 1, further comprising an extensible member extending from the frame member, the extensible member having a coupler at an end thereof.

## Patentansprüche

1. Vorrichtung (10) zum Verschluss eines persistierenden Foramen ovale eines Patienten, wobei die Vorrichtung gegenüberliegende axiale Enden hat, mit
einem Rahmenelement (12) mit einem zusammengeklappten Zustand und einem expandierten Zustand, in dem das Rahmenelement planar ist, wobei das Rahmenelement ein Paar länglicher Glieder (14, 16) umfasst, die jeweils ein erstes und ein zweites axiales Ende definieren und in der Nähe der ersten axialen Enden (18) an einem distalen Verbindungspunkt verbunden sind und in der Nähe der zweiten axialen Enden (18) an einem proximalen Verbindungspunkt verbunden sind, wobei der Rahmen derart ist, dass die länglichen Glieder im Wesentlichen einander benachbart positioniert sind, wenn das Rahmenelement im zusammengeklappten Zustand ist, und so positioniert sind, dass dazwischen eine Öffnung definiert ist, wenn das Rahmenelement im expandierten Zustand ist, wobei das Rahmenelement so bemessen und angeordnet ist, dass es perkutan in einen Bereich des Foramen ovale einführbar ist, wenn es im zusammengeklappten Zustand ist, und im Wesentlichen das Foramen ovale überspannt, wenn es im expandierten Zustand ist,
einem vom Rahmenelement getragenen Promoter (20, 27), der die biologische Entstehung fördert, um einen Verschluss des Foramen ovale zu bewirken, und
einem Verankerungsglied (22, 62) an jedem axialen Ende der länglichen Glieder, das so konfiguriert ist, dass es das Rahmenelement an dem Rahmenelement benachbartem Gewebe verankert, wenn das Rahmenelement im expandierten Zustand ist.

2. Vorrichtung nach Anspruch 1, wobei der Promoter ein Abdeckungsmaterial umfasst, wobei ein erster Abschnitt des Abdeckungsmaterials mit einem der länglichen Glieder in Eingriff steht und ein zweiter Abschnitt des Abdeckungsmaterials mit dem anderen länglichen Glied in Eingriff steht, wobei das Abdeckungsmaterial so positioniert ist, dass es die Öffnung im Wesentlichen überspannt, wenn das Rahmenelement im expandierten Zustand ist.

3. Vorrichtung nach Anspruch 2, wobei das Abdeckungsmaterial eine Zusammensetzung umfasst, die Gerinnselbildung im Foramen ovale fördern kann.

4. Vorrichtung nach Anspruch 3, wobei die gerinnselfördernde Zusammensetzung ein fasriges Material umfasst.

5. Vorrichtung nach Anspruch 4, wobei das fasrige Material Polyester- oder Seidenfasern umfasst.

6. Vorrichtung nach Anspruch 2, wobei das Abdeckungsmaterial eine Zusammensetzung umfasst, die das Gewebewachstum fördert und das Wachstum von dem Foramen ovale benachbartem Gewebe fördern kann.

7. Vorrichtung nach Anspruch 6, wobei die Zusammensetzung, die das Gewebewachstum fördert, Dünndarm-Submucosa und/oder ein Material auf Collagenbasis umfasst.

8. Vorrichtung nach Anspruch 1, wobei das Verankerungsglied mindestens einen Haken umfasst, der sich im benachbarten Gewebe verankern kann.

9. Vorrichtung nach Anspruch 1, wobei das Verankerungsglied mindestens eine Spule umfasst, die zur Verankerung der Vorrichtung im Foramen ovale bemessen und konfiguriert ist.

10. Vorrichtung nach Anspruch 9, wobei die Spule mehrere gerinnselfördernde Fasern aufweist, die damit in Eingriff stehen.

11. Vorrichtung nach Anspruch 1, ferner mit mindestens einem röntgendichten Marker, der mit dem Rahmenelement in Eingriff steht.

12. Vorrichtung nach Anspruch 1, wobei der Promoter mehrere gerinnselfördernde Fasern umfasst, die mit den Rahmenelementen in Eingriff stehen.

13. Vorrichtung nach Anspruch 12, wobei die Fasern mit mindestens einem der Rahmenelemente zwischen den Verbindungspunkten in Eingriff stehen.

14. Vorrichtung nach Anspruch 1, ferner mit einem ausfahrbaren Glied, das sich vom Rahmenelement erstreckt und an einem seiner Enden eine Kopplungsvorrichtung hat.

## Revendications

1. Dispositif (10) de fermeture d'un foramen ovale perméable d'un patient, le dispositif présentant des extrémités axiales opposées, comprenant:
un élément de cadre (12) présentant une condition écrasée et une condition déployée, dans laquelle l'élément de cadre est plan, l'élément de cadre comprenant une paire d'éléments allongés (14, 16) qui définissent chacun une première et une deuxième extrémités axiales, lesdits éléments allongés étant joints à proximité desdites première extrémités axiales (18) à un point de jonction distal et à proximité desdites deuxièmes extrémités axiales (18) à un point de jonction proximal, ledit cadre étant conçu de telle sorte que lesdits éléments allongés soient positionnés sensiblement adjacents l'un à l'autre lorsque l'élément de cadre se trouve dans la condition écrasée, et soient positionnés de telle sorte qu'une ouverture soit définie entre ceux-ci lorsque l'élément de cadre se trouve dans la condition déployée, l'élément de cadre étant dimensionné et agencé de manière à pouvoir être inséré de façon percutanée dans une région du foramen ovale lorsqu'il se trouve dans la condition écrasée, et à recouvrir sensiblement le foramen ovale lorsqu'il se trouve dans la condition déployée;
un promoteur (20, 27) porté par l'élément de cadre, le promoteur étant capable de promouvoir une formation biologique pour effectuer une fermeture du foramen ovale; et
un élément d'ancrage (22, 62) à chaque extrémité axiale des éléments allongés, configuré pour ancrer l'élément de cadre à un tissu adjacent à l'élément de cadre lorsque l'élément de cadre se trouve dans la condition déployée.

2. Dispositif selon la revendication 1, dans lequel ledit promoteur comprend une matière de recouvrement, une première partie de la matière de recouvrement étant engagée avec un des éléments allongés et une deuxième partie de la matière de recouvrement étant engagée avec l'autre élément allongé, la matière de recouvrement étant positionnée de manière à recouvrir sensiblement l'ouverture lorsque l'élément de cadre se trouve dans la condition déployée.

3. Dispositif selon la revendication 2, dans lequel la matière de recouvrement comprend une composition capable de promouvoir la formation de caillots dans le foramen ovale.

4. Dispositif selon la revendication 3, dans lequel la composition promotrice de caillots comprend une matière fibreuse.

5. Dispositif selon la revendication 4, dans lequel la matière fibreuse comprend des fibres de polyester ou de soie.

6. Dispositif selon la revendication 2, dans lequel la matière de recouvrement comprend une composition promotrice de croissance de tissu qui est capable de promouvoir la croissance d'un tissu adjacent au foramen ovale.

7. Dispositif selon la revendication 6, dans lequel la composition promotrice de croissance de tissu comprend au moins soit une sous-muqueuse du petit intestin, soit une matière à base de collagène.

8. Dispositif selon la revendication 1, dans lequel l'élément d'ancrage comprend au moins un crochet qui est capable de s'ancrer au tissu adjacent.

9. Dispositif selon la revendication 1, dans lequel l'élément d'ancrage comprend au moins une bobine dimensionnée et configurée pour ancrer le dispositif dans le foramen ovale.

10. Dispositif selon la revendication 9, dans lequel ladite bobine comprend une pluralité de fibres promotrices de caillots engagées dans celle-ci.

11. Dispositif selon la revendication 1, comprenant en outre au moins un marqueur radio-opaque engagé avec l'élément de cadre.

12. Dispositif selon la revendication 1, dans lequel le promoteur comprend une pluralité de fibres promotrices de caillots engagées avec les éléments de cadre.

13. Dispositif selon la revendication 12, dans lequel les fibres sont engagées avec au moins un des éléments de cadre entre lesdits points de jonction.

14. Dispositif selon la revendication 1, comprenant en outre un élément extensible qui s'étend à partir de l'élément de cadre, l'élément extensible présentant un coupleur à une extrémité de celui-ci.
